# EUROPEAN PATENT SPECIFICATION

(11) **EP 1 502 578 B1**
(45) Date of publication and mention of the grant of the patent: **15.04.2009**
(21) Application number: 03017007.0
(22) Date of filing: 26.07.2003
(51) Int. Cl.: A61K 8/81, A61Q 5/10, A61Q 5/12

(54) **Hair coloring conditioning composition**
Konditionierende Haarfärbezusammensetzung
Compostion de coloration et de conditionnement capillaire

(43) Date of publication of application: 02.02.2005
(73) Proprietor: KPSS-Kao Professional Salon Services GmbH, 64297 Darmstadt (DE)
(72) Inventor: Hoffmann, Martin, 64673 Zwingenberg (DE); Bistram, Vera, 64683 Einhausen (DE)

(56) References cited:
- EP-A- 0 960 617
- EP-A- 1 127 566
- EP-A- 1 269 975
- WO-A-02/057322
- DE-A- 10 101 946
- DE-A- 19 751 550

## Description

The present invention relates to a transparent gel type coloring hair conditioner composition showing relatively low viscosity changes at fluctuating temperatures and comprising at least one cationic polymer as a thickener and stabilizer and at least one direct acting dyestuff and free from fatty alcohol, and, therefore, not in the emulsion form which is especially excellently suitable for rinse off application as well as leave in application. The colouring conditioner composition of the present invention preferably further comprises at least one solubilizer selected from non-ionic, cationic and/or amphoteric surfactants. Furthermore, the colouring conditioners of the present invention include active ingredients selected from hair restructuring ingredients, moisturising ingredients and natural plant extracts showing hair conditioning and/or restructuring benefits.

Hair conditioners of various types either in application or in the form of preparation types have found their wide range of usage in hair dressing practice. Among those rinse off types of conditioners have always been preferred in hair care. Usually, rinse-off type conditioners are in the form of emulsions and comprising fatty alcohols and emulsifiers of different character as the main principal ingredients. In addition, they certainly comprise conditioning ingredients of various types and those of common ingredients in cosmetic preparations. A general overview on the known hair conditioning products and also their usual compositions can be found in the monography of K. Schrader, "Grundlage und Rezepturen der Kosmetika", 2nd Ed. 1989, pp 722 - 781. Those have also been used as colouring and /or colour enhancing conditioners.

Colouring conditioners have also been known for a long time. Those are mainly, as the ones without dyestuff, in an emulsion form and contain those fatty alcohol and emulsifiers as the main ingredients. EP 1 127 566 A2 discloses colouring conditioners based on a cationic polymer and direct dyes. The cationic polymer disclosed is polyquaternium 37. As all the compositions disclosed are produced with a raw material containing only 50% polyquaternium 37 in a dispersing medium, all are non transparent compositions.

The conditioners are, in general as well coloring ones, non-transparent preparations and often packed into a non transparent packaging as well and, therefore, lacking the attractiveness, based on appearance judgement, to the consumer. Furthermore, those conventional conditioners show large fluctuations in their consistency (viscosity) to temperature changes. This is certainly a disadvantage as products of same type are marketed in large number of countries with variable climate conditions. Consequently, formulation and/or production procedure adjustments are very much desirable, though only for consistency, when marketing the product in countries with variable climate conditions.

The desire to transparent gel type of conditioners is there as attractive appearance, even only given by the transparent appearance, is one of the main attributes to gain more customers. In addition, the cosmetic products as such decorated with colourful label designs improve further the attractiveness especially when packed into a transparent packaging.

Gel type preparations are usually found not to be suitable for rinse off applications as after rinsing off, the hair is usually not combable, has dull appearance and is not manageable at all.

Those problems are solved by the coloring conditioner composition of claim 1. The conditioner has a consistency which is less sensitive to temperature fluctuations and furthermore especially excellently suitable for rinse off applications as well as leave in. The conditioner preparations of the present invention may further comprise at least one solubilizer selected from non-ionic, cationic and/or amphoteric surfactants. Furthermore, the conditioners of the present invention can include active ingredients selected from hair restructuring ingredients, moisturising ingredients and natural plant extracts showing hair conditioning and/or restructuring benefits.

The colouring conditioners of the present invention make hair easy to comb and improve shine, elasticity and volume and particularly improve structure of those damaged, chemically processed hair, which is known under the concept "hair repair' together with colour enhancing.

The transparency of the colouring conditioner compositions of the present invention is judged macroscopically by naked eye thorough a bottle which has a thickness of less than 5 cm. In the case that the dyestuff content is high, the thickness of the bottle can be reduced to 1 cm. The bottle can be preferably in round shaped or in an oval shape. Although the inventors of the present invention judge the transparency of the conditioners macroscopically, certainly there is a possibility to evaluate the transparency by, for example, spectrophotometrically. In this case, the transmitted light of the sample is measured against a transparent preparation, which is usually water. The measurement wavelength should be chosen in the visible range and errors that may occur due to light absorbance of substances included in the conditioner should be taken into account.

The cationic polymer used in the compositions of the present invention is known as Polyquaternium 37 with its CTFA adopted name. Chemically, it is homopolymer of dimethylaminoethyl methacrylate quartemized with methyl chloride. The same structure is available from Ciba Chemicals under the trade name Salcare SC95 and 96. However, those two materials are delivered in suspension form either as dispersed in an oily medium or in a non-ionic surfactant mixture. Recently, from Cosmetic Rheologies Ltd., it has been possible to receive the polymer in a powder form which, first of all, does not contain any additional raw materials and dissolves easily in water and forms gel. It is marketed with the trade name Ultragel 300.

Unlike the other polymers known with the same CTFA adopted name, this is nearly pure raw material in powder form, it may certainly contain some impurities which do not disturb preparation of the conditioners of the present invention. Since the others, Salcare SC95 and 96 are both in a mixture with additional ingredients, it is not possible with those to prepare transparent gel type hair conditioners. Furthermore, the conditioners prepared with those also do not show good hair conditioning properties when used as a rinse of conditioner. In other words, opposite to the ones prepared with the powder Polyquaternium 37 nearly pure, those conditioners thickened with Salcare types do not improve hair quality in terms of combability, elasticity, manageability, volume and body and shine when used as a rinse off conditioner.

The conditioners of the present invention contain polyquatemium 37 known with the trade name Ultragel 300 at a concentration of 0.01 to 5%, preferably, 0.01 to 3.5% and more preferably 0.05 - 2.5% by weight calculated to the total composition.

As direct dyes, cationic dyes are useful. The use of anionic dyes is impossible as those are incompatible with the cationic thickener used according to the invention and destroy the gel structure. The concentration of those dyes is in between 0.001 - 5% by weight, preferably 0.001 -2.5% by weight and more preferably 0.001 - 1 % by weight calculated to the total composition.

The useful cationic dyes, especially preferred in the colouring conditioners according to the invention are:

| | |
|---|---|
| Basic Blue 6, | C.I.-No. 51,175; |
| Basic Blue 7, | C.I.-No. 42,595; |
| Basic Blue 9, | C.I.-No. 52,015; |
| Basic Blue 26, | C.I.-No. 44,045; |
| Basic Blue 41, | C.I.-No. 11,154; |
| Basic Blue 99, | C.I.-No. 56,059; |
| Basic Brown 4, | C.I.-No. 21,010; |
| Basic Brown 16; | C.I.-No. 12,250; |
| Basic Brown 17, | C.I.-No. 12,251; |
| Natural Brown 7, | C.I.-No. 75,500; |
| Basic Green 1, | C.I.-No. 42,040; |
| Basic Red 2, | C.I.-No. 50,240; |
| Basic Red 22, | C.I.-No. 11,055; |
| Basic Red 76, | C.I.-No. 12,245; |
| Basic Violet 1, | C.I.-No. 42,535; |
| Basic Violet 3, | C.I.-No. 42,555; |
| Basic Violet 10, | C.I.-No. 45,170; |
| Basic Violet 14, | C. I.-No. 42,510; |
| Basic Yellow 57, | C.I.-No. 12,719. |
| HC Blue No.2 | |
| HC Blue No.4 | |
| HC Blue No.5 | |
| HC Blue No.6 | |
| HC Blue No.7 | |
| HC Blue No.8 | |
| HC Blue No.9 | |
| HC Blue No.10 | |
| HC Blue No.11 | |
| HC Blue No.12 | |
| HC Blue No.13 | |
| HC Brown No.1 | |
| HC Brown No.2 | |
| HC Green No.1 | |
| HC Orange No.1 | |
| HC Orange No.2 | |
| HC Orange No.3 | |
| HC Orange No.5 | |
| HC Red BN | |
| HC Red No.1 | |
| HC Red No.3 | |
| HC Red No.7 | |
| HC Red No.8 | |
| HC Red No.9 | |
| HC Red No.10 | |
| HC Red No.11 | |
| HC Red No.13 | |
| HC Red No.14 | |
| HC Violet BS | |
| HC Violet No.1 | |
| HC Violet No.2 | |
| HC Yellow No.2 | |
| HC Yellow No.4 | |
| HC Yellow No.5 | |
| HC Yellow No.6 | |
| HC Yellow No.7 | |
| HC Yellow No.8 | |
| HC Yellow No.9 | |
| HC Yellow No. 10 | |
| HC Yellow No. 11 | |
| HC Yellow No.12 | |
| HC Yellow No.13 | |
| HC Yellow No.14 | |
| HC Yellow No. 15 | |

The coloring conditioners of the present invention comprise in addition to the cationic polymer one or more cationic surfactants as conditioner and solubilizers presented with the general formula 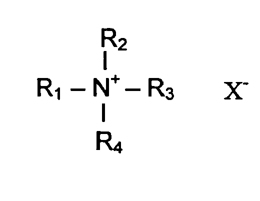 where R₁ is a saturated or unsaturated, branched or non-branched alkyl chain with 8-22 C atoms or

R₅ CO NH (CH₂)ₙ

where R₅ is saturated or unsaturated, branched or non-branched alkyl chain with 7-21 C atoms and n has value of 1 - 4, or

R₆ CO O (CH₂)ₙ

where R₆ is saturated or unsaturated, branched or non-branched alkyl chain with 7-21 C atoms and n has value of 1 - 4, and
R₂ is H or unsaturated or saturated, branched or non-branched alkyl chain with 1 - 4 C atoms or

R₅ CO NH (CH₂)ₙ

or

R₆ CO O (CH₂)ₙ

where R₅ , R₆ and n are same as above.

R₃ and R₄ are H or lower alkyl chain with 1 to 4 Carbon atoms, and X is chloride, bromide, methosulfate.

Typical examples of those ingredients are cetyl trimethly ammonium chloride, stear trimonium chloride, behentrimoinium chloride, stearamidopropyl trimonuim chloride, dioleoylethyl dimethyl ammonium methosulfate, dioleoylethyl hydroxyethylmonium methosulfate.

The concentration of cationic surfactants typically is from 0.01 to 10%, preferably 0.01 - 5%, more preferably 0.05 - 5% and most preferably 0.05 - 3.5% by weight, calculated to the total composition.

Furthermore, conditioners of the present invention comprise one or more non-ionic surfactants as solubilizers. Suitable nonionic surfactants are compounds from the category of alkyl polyglucosides with the general formula

R₇-O-(CH₂CH₂O)ₙ-Zₓ,

wherein R₇ is an alkyl group with 8 to 20, preferably 10 to 14 carbon atoms, Zₓ is a saccharide group with 5 to 6 carbon atoms, n stands for a number from 0 to 10, and x is a number between 1 and 5, preferably 1.1 to 2.5.

Further nonionic surfactants useful in the compositions according to invention are C₁₀-C₂₂-fatty alcohol ethoxylates. Especially suited C₁₀-C₂₂-fatty alcohol ethers are the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

Other additionally useful surfactants are, for example, the various sorbitan esters, such as polyethylene glycol sorbitan stearic acid ester, fatty acid polyglycol ester or also mixed condensates of ethylene oxide and propylene oxide, as they are on the market, for example, under the trade name "Pluronics^{R"}.

Hydrogenated castor oil with variable ethylene glycol units is found to be suitable non-ionic solubilizers. Those are for example known from BASF under the trade name Cremophor.

Further additionally useful surfactants are amineoxides.
Such amineoxides are state of the art, for example C₁₂-C₁₈- alkyl dimethyl amineoxides such as lauryl dimethyl amineoxide, C₁₂-C₁₈- alkyl amidopropyl or
ethyl amineoxides, C₁₂-C₁₈ -alkyl di(hydroxyethyl) or (hydroxypropyl) amineoxides, or also amineoxides with ethylene oxide and/or propylene oxide groups in the alkyl chain.

Suitable amineoxides are on the market, for example, under the trade names "Ammonyx® ", "Aromox®" or "Genaminox®".

The concentration of nonionic surfactants is typically from 0.01 to 10%, preferably 0.05 - 7.5%, more preferably 0.1 - 5% and most preferably 0.1 - 3.5% by weight, calculated to the total composition.

Further optional surfactant components are fatty acid mono- and dialkanolamides, such as coco fatty acid monoethanolamide and myristic fatty acid monoisopropanolamide.

Conditioners of the present invention can contain amphoteric or zwitterionic surfactants as solubilizing agents. Suitable ones are in particular the various known betaines such as fatty acid amidoalkyl betaines and sulfobetaines; for example lauryl hydroxy sulfobetaine; long-chain alkyl amino acids, such as cocoaminoacetate, cocoaminopropionate and sodium cocoamphopropionate and -acetate have also proven suitable.

In detail it is possible to use betaines of the structure 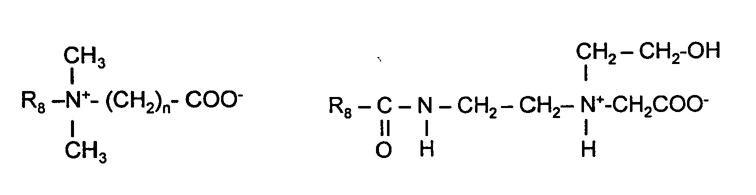 wherein R₈ is a C₈-C₁₈-alkyl group and n is 1 to 3,
sulfobetaines of the structure 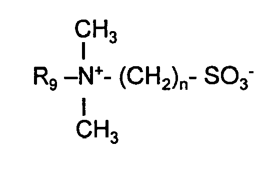 wherein R₉ is a C₈-C₁₈-alkyl group and n is 1 to 3,
and amidoalkyl betaines of the structure 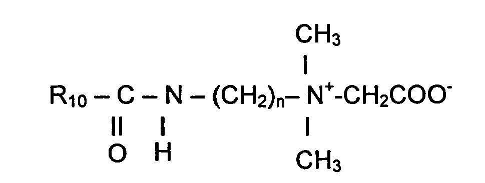 wherein R₁₀ is a C₈-C₁₈-alkyl group and n is 1 to 3,

Preferred are fatty acid amidoalkyl betaines, in particular cocoamidopropyl betaine, and cocoamphoacetate and -propionate, in particular the sodium salts thereof. The concentration can be typically less than 5%, preferably less than 3.5% by weight calculated to the total composition.

Solubilizers of nonionic, cationic and amphoteric character can certainly be used in combination in the conditioners of the present invention. In this case, the total concentration of the solubilizer in the conditioner should, as a rule, not exceed 15%, prefereably 10% and more preferably 7.5% by weight calculated to the total composition. It should be noted that the solubilizers preferred are nonionic and cationic surfactants.

Colouring conditioner compositions of the present invention can contain additional cationic polymers as conditioning agents. Suitable polymers are those of cationc polymers best known with their CTFA category name Polyquaternium. Typical examples of those Polyquatemium 6, Polyquatemium 7, Polyquaternium 10, Polyquaternium 11, Polyquatemium 16, Polyquatemium 22 and Polyquaternium 28. It has been found out that especially those of cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride, are preferred ones. Furthermore, chitosan and chitin can also be included in the compositions as cationic natural polymers.

Typical concentration range for any of the conditioners mentioned above can be 0.01 - 5% by weight, preferably 0.01 - 3.5% by weight and more preferably 0.05 - 2% by weight calculated to the total composition.

The colouring conditioner compositions may contain organic solvents, for example, as penetration enhancer such as ethanol. propanol, isopropanol, benzyl alcohol, benzyloxyethanol, alkylene carbonates such as ethylene carbonate and propylene carbonate, phenoxyethanol, butanol, isobutanol, cyclohexane, cyclohexanol, hexyleneglycol, ethylene glycol, butylenes glycol, propylene glycol, benzyl glycol, ethylene glycol monoethylether, ethylene glycol monobutyl ether, ethylene glycol monophenyl ether, 1-phenylethylalcohol, 2-phenylethylalcohol, o-methoxyphenol.

Concentration of organic solvents in the conditioner composition can be in the range from 0.1 to 10% by weight, preferably 0.1 to 7.5% by weight, and more preferably 0.1 to 5% by weight calculated to the total composition. The organic solvents may at the same time serve to solubilize ingredients which are not readily soluble in the conditioner composition.

Conditioner compositions of the present invention can optionally contain non-ionic polymers such as hydroxyethylcellulose, hydroxypropylclellulose, xanthan gum, xyloglucan, polyvinylalcohol, polyvinylpyrrolidone or their derivatives.

Anionic polymers should not be used in the conditioner compositions of the present invention as incompatibilities arose with the main thickening cationic polymer and other cationic conditioners. Typical example of those should not be used is acrylate type of polymers know with the trade name Carbopol from Goodrich.

The conditioner compositions can have viscosity values between 1,000 mPa.s to 70,000 mPa.s, preferably 1,000 mPa.s to 50,000 mPa.s and more preferably 1,000 mPa.s to 40,000 mPa.s measured at 20°C with Brookfield viscosimeter with, for example, Spindle 5 at 5 rpm. The viscosity values are read after 30 seconds from the start of the measurement. In the selection of the viscosity, special attention must be paid to the way of application and packaging to be used. It should be noted that the viscosity of the conditioners is less sensitive to temperature fluctuations. In other words, the viscosity changes observed with either decreasing or increasing temperatures is relatively small when compared to well known gel type of preparations thickened such as with hydroxyethylcellulose.

The pH of the conditioners of the present invention varies from 2 to 7, particularly 2
to 6 and more particularly 2.5 to 6.0. Moreover, the optimum conditioning properties observed with the compositions having pH at 5.0±0.5.

For adjusting the pH of the said conditioner compositions, following ingredients can be used: Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, salicylic acid or acetic acid or inorganic acids such as hydrochloric acid, phosphoric acid, sulphuric acid, nitric acid. Concentration of the organic and/or inorganic acids or their mixtures should be adjusted in a way that conditioner composition so obtained has a pH value between 2 to 7. Typically concentration for acids can be 0.01 - 5% by weight, preferably 0.01 - 4% by weight, more preferably 0.05 - 2.5% by weight calculated to the total composition. The pH of the conditioner composition can also be adjusted to the required pH by using alkaline solution such as sodium hydroxide, potassium hydroxide or their salts with those acids mentioned above in the case that at the selected acid concentration pH of the composition is lower than that of the aimed value.

The conditioner composition may contain active ingredients selected from moisturisers, hair restructuring agents and natural ingredients showing conditioning benefits.

The moisturising agents are selected from panthenol, polyols, such as glycerol, polyethylene glycols with molecular weight 200 to 20,000. The moisturising ingredients can be included in the conditioner compositions at a concentration range of 0.01 - 2.5% by weight calculated to the total composition.

Hair restructuring agents preferred are especially the ones disclosed in the German patent DE 197 51 550 C2.

One of the known hair restructuring agents is ceramide type of compound with the general formula 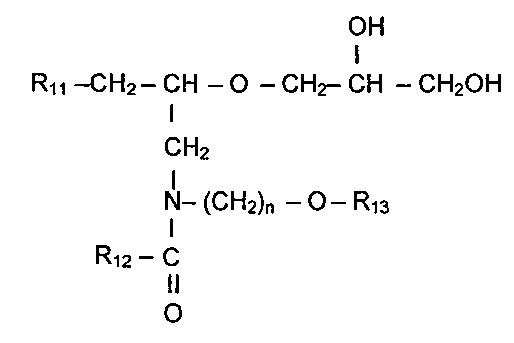 where R¹¹ and R¹² are independent from each other alkyl- or. alkenyl group mit 10 to 22 carbon atoms, R¹³ is methyl, ethyl, n-propyl or isopropyl group and n is a number between 1 to 6, preferably 2 or 3.

Other preferred hair restructuring agents are fatty acids with 10 to 24 carbon atoms and especially with 16 to 24 carbon atoms.

Sterols,especially the phytosterols, are as well preferred hair restructuring agents as disclosed in the above mentioned german patent. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

The concentration of ceramide in the conditioners of the present invention is in the range of 0.01 to 2 and especially 0.01 to 1% by weight calculated to the total weight of the composition. The fatty acids is contained at a level of 0.01 to 2.5% and epecially 0.01 to 1% by weight calculated to the total weight of the composition. Phytosterol concentration of the conditioners is less than 1% and preferably less than 0.5% by weight calculated to the total weight of the composition. It should be noted that the combination of those three ingredients are preferably used in combination in the composition of the present invention. Special attention has to be paid when formulating such compositions to the transparency of the compositions. This can be achieved by carefully adjusting the solubilizers concentration in the compositions.

Natural plant extracts showing hair conditioning and/or restructuring effects can be used in the conditioners. Those are preferably the extracts from almond, Aloe Vera, coconut, mango, peach, lemon, wheat, rosemary, apricot, algae, grapefruit, sandalwood, lime and orange. Those extracts used are the ones commercially available and generally include organic solvents such as propylene glycol, butylenes glycol, ethanol, isopropanol. The active matter in those extracts can vary largely, i.e. in the range of 1 - 30% by weight. Concentration of those as an extract depending on the compatibility with the other ingredients can be in the range of 0.1 to 5%, preferably 0.1 to 2% by weight calculated to the total composition.

The following examples are to illustrate the invention, but not limiting it.

### Example 1 (Reference example)

All values are in % by weight

| | |
|---|---|
| Arianor sienna brown | 0.01 |
| Arianor straw yellow | 0.01 |
| Ultragel 300 | 1.00 |
| Merquat 550 | 2.00 |
| Panthenol | 0:50 |
| Hydroxyethylcellulose | 0.20 |
| Glycerin | 2.00 |
| Cetrimonium chloride | 1.00 |
| Lactic acid | 0.07 |
| Fragrance | q.s. |
| Preservative | q.s. |
| Water | to 100 |

Colouring conditioner is prepared by dissolving Ultragel 300 in water first and other ingredients are added one by one to the gel solution. In the case of the solid ingredient, such as dyes, it is preferable either alone or combined with others to prepare their solution first in a small portion of water. The colouring conditioner thus obtained has a pH value of 4 and has a viscosity of 17,400 mPa.s measured with Brookfield viscosimeter at 20°C with spindle 4 at 10 rpm. The colouring conditioner so obtained is transparent.

Gold blond hair color is obtained when applied onto hair either rinse-off or leave in.

For illustration of the insensitivity of the viscosity of the composition to the temperature fluctuations, viscosity of the above example is measured at various temperature. At the same time, a new composition is produced in which only hydroxyethylcellulose is used as thickener, so that one of the compositions produced is containing 1.2% hydroxyethylcellulose (HEC-1.2%). In order to illustrate that the viscosity is in the same range as in the inventive composition, the hydroxyethylcellulose concentration is increased to 1.7% by weight calculated to the total composition (HEC-1.7%) . The viscosity results are presented in Table I.

**Table I**

| **Viscosity mPa.s** | | | |
|---|---|---|---|
| Temperature °C | Example 1 | HEC-1.2% | HEC-1.7% |
| 5 | 18040 | 6719 | 18720 |
| 20 | 17400 | 3720 | 13500 |
| 35 | 16380 | 2160 | 7500 |
| 50 | 15780 | 1240 | 4760 |

Viscosity values are measured with Brookfiled viscosimeter at given temperature, with spindle 4 and at 10 rpm.

As obvious from the results in Table I, viscosity of the conditioners thickened with hydroxyethylcellulose is strongly dependent on temperature whereas the viscosity of the inventive composition thickened with Polyquaternium 37 (Ultragel 300) changes slightly.

In order to show the conditioning effect of the inventive composition in rinse off application, the composition of example 1 is tested against a composition of the following formula. In the comparative example, polyquaternium 37 is excluded, the concetration of Hydroxy ethyl cellulose is increased to 1.7% to thicken, and as a conditioner Merquat 550 concentration is increased to 14.5% raw material which corresponds to 1.16% by weight cationic conditioning ingredient.

### Example 2 (comparative formula, not inventive)

All values are in % by weight.

| | |
|---|---|
| Arianor sienna brown | 0.01 |
| Arianor straw yellow | 0.01 |
| Merquat 550 | 14.50 |
| Panthenol | 0.50 |
| Hydroxyethylcellulose | 1.70 |
| Glycerin | 2.00 |
| Cetrimonium chloride | 1.00 |
| Lactic acid | 0.07 |
| Fragrance | q.s. |
| Preservative | q.s. |
| Water | to 100 |

Both compositions (inventive and comparative) are tested against each other in a half-head test on 10 women. For this purpose whole head of test person is washed with a Shine and Vitality shampoo (commercially available product under the brand name Goldwell Definition) and the equal amount from both conditioners are applied to each side and processed for 5 min. Subsequently rinsed off and towel dried and further dried with a dryer. In wet and dry stage sensory evaluations are made on the hair properties given in Table II.

**Table 11:Sensory evaluation**

| | Prefered | | | |
|---|---|---|---|---|
| | Parameter | Example 1 | Example 2 | no preference |
| | | | | |
| Wet | Easy combing | 7 | 2 | 1 |
| | Smoothness | 6 | 2 | 2 |
| | Roughness | 2 | 2 | 6 |
| | | | | |
| Dry | Easy combing | 7 | 2 | 1 |
| | Smoothness | 6 | 2 | 2 |
| | Elasticity | 8 | 2 | 0 |
| | Volume | 4 | 5 | 1 |
| | Shine | 8 | 1 | 1 |
| | Hair color | 9 | 0 | 1 |

Comments from the test persons on the color appareance were that the side where inventive composition was applied more intensive and brilliant colour.

Similar results are obtained with the following inventive examples.

### Example 3

All values are in % by weight.

| | |
|---|---|
| Arianor madder red | 0.08 |
| Arianor mahagony | 0.10 |
| Basic Violet 2 | 0.008 |
| Ultragel 300 | 1.20 |
| Panthenol | 0.50 |
| Decyl polyglucoside | 3.50 |
| Ceramide of the formula | 0.20 |
| Palmitic acid | 0.10 |
| Avocadin | 0.05 |
| Benzyloxyethanol | 2.00 |
| Glycerin | 2.00 |
| Behentrimoniumchloride | 2.00 |
| Citric acid | q.s. to pH 4.6 |
| Fragrance | q.s. |
| Preservative | q.s. |
| Water | to 100 |

Formulation is prepared as described for example 1 except ceramide and palmitic acid are first dissolved in decyl polyglucoside and than added to the rest of the composition. The composition so obtained is clear, dark red and has a viscosity around 19,500 mPa.s measured with Brookfield viscosimeter at 20°C with spindle 4 at 10 rpm. The composition is especially suitable for damaged hair as restructurant, repair treatment as well. Upon application red hair colour is obtained. It should be noted that the colour intensity obtained is very much dependent on the dyestuff concentration. The colour obtained with the above conditioner is a red touch on the base colour.

### Example 4 (Reference example)

All values are as % by weight.

| | |
|---|---|
| Arianor sienna brown | 0.05 |
| Arianor mahagony | 0.026 |
| Arianor steel blue | 0.02 |
| Ultragel 300 | 1.20 |
| Panthenol | 0.70 |
| PEG-60 hydrogenated castor oil | 0.50 |
| Aloe vera extract | 1.00 |
| Polyethylene glycol 200 | 1.00 |
| Stearamidopropylamine | 2.00 |
| Citric acid | q.s. to pH 3.5 |
| Fragrance | q.s. |
| Preservative | q.s. |
| Water | to 100 |

Formulation is prepared as described for example 1 The composition so obtained is clear and has a viscosity around 23,000 mPa.s measured with Brookfield viscosimeter at 20°C with spindle 4 at 10 rpm. Upon application a deep brown touch is obtained. The composition is especially suitable for dry hair as a moisturising conditioner as well.

### Example 5 (Reference example)

All values are as % by weight.

| | |
|---|---|
| Arianor sienna brown | 0.08 |
| Arianor mahagony | 0.05 |
| Ultragel 300 | 1.20 |
| Panthenol | 0.70 |
| PEG-60 hydrogenated castor oil | 0.50 |
| Sandelwood extract | 1.00 |
| Polyethylene glycol 200 | 1.00 |
| Stearamidopropylamine | 2.00 |
| Citric acid | q.s. to pH 4.5 |
| Fragrance | q.s. |
| Preservative | q.s. |
| Water | to 100 |

Formulation is prepared as described for example 1 The composition so obtained is clear and has a viscosity around 23,000 mPa.s measured with Brookfield viscosimeter at 20°C with spindle 4 at 10 rpm. Upon application a copper color is obtained. The composition is especially suitable for dry hair as a moisturising conditioner as well.

### Example 6 (Reference example)

All values are as % by weight.

| | |
|---|---|
| Arianor madder red | 0.0001 |
| Basic violet 2 | 0.000045 |
| Arianor steel blue | 0.002 |
| Ultragel 300 | 1.20 |
| Panthenol | 0.70 |
| PEG-60 hydrogenated castor oil | 0.50 |
| Aloe vera extract | 1.00 |
| Polyethylene glycol 200 | 1.00 |
| Stearamidopropylamine | 2.00 |
| Citric acid | q.s. to pH 5.2 |
| Fragrance | q.s. |
| Preservative | q.s. |
| Water | to 100 |

Formulation is prepared as described for example 1 The composition so obtained is clear and has a viscosity around 23,000 mPa.s measured with Brookfield viscosimeter at 20°C with spindle 4 at 10 rpm. Platin blond appearance is observed uipon application on a gray hair. The composition is as well applied to bleached hair and anti yellow effect is observed. The composition is especially suitable for dry hair as a moisturising conditioner as well.

### Example 7

All values are in % by weight.

| | |
|---|---|
| HC Red No. 3 | 0.08 |
| Arianor mahagony | 0.10 |
| Basic Violet 2 | 0.008 |
| Ultragel 300 | 1.20 |
| Panthenol | 0.50 |
| Decyl polyglucoside | 3.50 |
| Ceramide of the formula | 0.20 |
| Palmitic acid | 0.10 |
| Avocadin | 0.05 |
| Benzyloxyethanol | 2.00 |
| Glycerin | 2.00 |
| Behentrimoniumchloride | 2.00 |
| Citric acid | q.s. to pH 4.6 |
| Fragrance | q.s. |
| Preservative | q.s. |
| Water | to 100 |

Formulation is prepared as described for example 1 except ceramide and palmitic acid are first dissolved in decyl polyglucoside and than added to the rest of the composition. The composition so obtained is clear, dark red and has a viscosity around 19,500 mPa.s measured with Brookfield viscosimeter at 20°C with spindle 4 at 10 rpm. The composition is especially suitable for damaged hair as restructurant, repair treatment as well. Upon application red hair colour is obtained. It should be noted that the colour intensity obtained is very much dependent on the dyestuff concentration. The colour obtained with the above conditioner is a red touch on the base colour.

### Example 8 (Reference example)

All values are in % by weight

| | |
|---|---|
| Arianor sienna brown | 0.01 |
| HC Yellow No. 5 | 0.01 |
| Ultragel 300 | 1.00 |
| Merquat 550 | 2.00 |
| Panthenol | 0.50 |
| Hydroxyethylcellulose | 0.20 |
| Glycerin | 2.00 |
| Cetrimonium chloride | 1.00 |
| Lactic acid | 0.07 |
| Fragrance | q.s. |
| Preservative | q.s. |
| Water | to 100 |

Colouring conditioner is prepared by dissolving Ultragel 300 in water first and other ingredients are added one by one to the gel solution. In the case of the solid ingredient, such as dyes, it is preferable either alone or combined with others to prepare their solution first in a small portion of water. The colouring conditioner thus obtained has a pH value of 4 and has a viscosity of 17,400 mPa.s measured with Brookfield viscosimeter at 20°C with spindle 4 at 10 rpm. The colouring conditioner so obtained-is-transparent.

Gold blond hair colour is obtained when applied onto hair either rinse-off or leave in.

## Claims

1. Transparent hair colouring conditioning agent **characterised in that** it comprises polyquaternium-37 as thickener and stabilizer, at least one direct acting cationic hair dye, as hair restructuring agents ceramide according to the formula and free from fatty alcohol so that it is not in the form of an emulsion, and all concentrations are calculated to the total composition.

2. Transparent hair colouring conditioning agent according to claim 1 **characterised in that** it comprises polyquaternium 37 as thickener and stabilizer at a concentration between 0.01 to 5% by weight calculated to the total composition.

3. Transparent hair colouring conditioning agent according to claims 1 and 2 **characterised in that** it comprises at least one direct acting cationic hair dye at a concentration between 0.001 to 5% by weight calculated to the total composition.

4. Transparent hair conditioning agent according to any of the preceding claims **characterised in that** it comprises solubilizers selected from non-ionic, cationic and amphoteric/zwitterionic surfactants.

5. Transparent hair conditioning agent according to any of the preceding claims **characterised in that** it further comprises active ingredients selected from hair restructuring ingredients, moisturising ingredients and natural plant extracts.

6. Transparent hair conditioning agent according to any of the preceding claims **characterised in that** it comprises organic solvents as solubilizer and/or penetration enhancer. 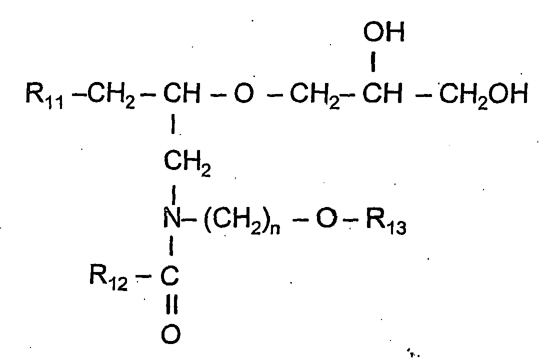 where R¹¹ and R¹² are independent from each other alkyl- or alkenyl group mit 10 to 22 carbon atoms, R¹³ is methyl, ethyl, n-propyl or isopropyl group and n is a number between 1 to 6, preferably 2 or 3 at a concentration of 0.01 -2% by weight, fatty acids with 10 to 24 carbon atoms at a concentration of 0.01-2.5% by weight and phytosterol.

7. Transparent hair conditioning agent according to any of the preceding claims **characterised in that** it comprises cationic polymers as additional conditioners.

8. Transparent hair conditioning agent according to any of the preceding claims **characterised in that** it comprises non-ionic polymers.

9. Transparent hair conditioning agent according to any of the preceding claims **characterised in that** it is free from any of anionic polymers.

10. Transparent hair conditioning agent according to any of the preceding claims **characterised in that** it comprises organic and/or inorganic acids and/or their mixtures and/or salts.

11. Transparent hair conditioning agent according to any of the preceding claims **characterised in that** it has a pH in the range of 2 to 7.

12. Transparent hair conditioning agent according to any of the preceding claims **characterised in that** it has a viscosity between 1,000 mPa.s to 70,000 mPa.s measured with Brookfield viscosimeter at 20°C with for example spindle 4 at 10 rpm.

## Patentansprüche

1. Transparentes Haar färbendes Konditionierungsmittel, **dadurch gekennzeichnet, dass** es Polyquaternium- 37 als Verdickungs- und Stabilisierungsmittel, mindestens eine direkt ziehende kationische Haarfarbe, als restrukturierende Wirkstoffe Ceramid entsprechend der Formel 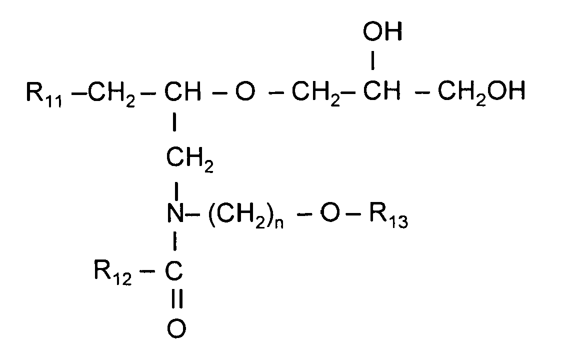 wobei R¹¹ und R¹² unabhängig voneinander Alkyl- oder AlkenylGruppen mit 10 bis 22 Kohlenstoffatomen sind, R¹³ eine Methyl-, Ethyl-, n-Propyl oder Isopropyl- Gruppe ist, und n eine Zahl zwischen 1 bis 6, vorzugsweise 2 oder 3 ist in einer Menge von 0.01 - 2 Gew.- %, Fettsäuren mit 10 bis 20 Kohlenstoffatomen in einer Menge von 0,01 - 2,5 Gew.- % und Phytosterol enthält,
und frei von Fettalkohol ist, so dass es nicht in Form einer Emulsion vorliegt und alle Mengenangaben auf die Gesamtzusammensetzung berechnet sind.

2. Transparentes Haar färbendes Konditionierungsmittel nach Anspruch 1, **dadurch gekennzeichnet, dass** es Polyquaternium- 37 als Verdickungs- und Stabilisierungsmittel in einer Menge zwischen 0,01 bis 5 Gew.- %, berechnet auf die Gesamtzusammensetzung, enthält.

3. Transparentes Haar färbendes Konditionierungsmittel nach den Ansprüchen 1 und 2, **dadurch gekennzeichnet, dass** es mindestens eine kationische direkt ziehende Haarfarbe in einer Menge zwischen 0,001 bis 5 Gew.- %, berechnet auf die Gesamtzusammensetzung, enthält.

4. Transparentes Haar färbendes Konditionierungsmittel nach einem der vorhergehenden Ansprüche, **dadurch gekennzeichnet, dass** es Lösungsvermittler, ausgewählt aus nichtionischen, kationischen und amphoterischen/zwitterionischen Tensiden enthält.

5. Transparentes Haar färbendes Konditionierungsmittel nach einem der vorhergehenden Ansprüche, **dadurch gekennzeichnet, dass** es weitere aktive Wirkstoffe, ausgewählt aus restrukturierenden Wirkstoffen, Feuchtigkeit spendenden Wirkstoffen und natürlichen Pflanzenextrakten enthält.

6. Transparentes Haar färbendes Konditionierungsmittel nach einem der vorhergehenden Ansprüche, **dadurch gekennzeichnet, dass** es organische Lösungsmittel als Lösungsvermittler und/oder Penetrationsverstärker enthält.

7. Transparentes Haar färbendes Konditionierungsmittel nach einem der vorhergehenden Ansprüche, **dadurch gekennzeichnet, dass** es kationische Polymere als zusätzliche Konditionierer enthält.

8. Transparentes Haar färbendes Konditionierungsmittel nach einem der vorhergehenden Ansprüche, **dadurch gekennzeichnet, dass** es nichtionische Polymere enthält.

9. Transparentes Haar färbendes Konditionierungsmittel nach einem der vorhergehenden Ansprüche, **dadurch gekennzeichnet, dass** es frei von anionischen Polymeren ist.

10. Transparentes Haar färbendes Konditionierungsmittel nach einem der vorhergehenden Ansprüche, **dadurch gekennzeichnet, dass** es organische und/oder anorganische Säuren und/oder deren Mischungen und/oder Salze enthält.

11. Transparentes Haar färbendes Konditionierungsmittel nach einem der vorhergehenden Ansprüche, **dadurch gekennzeichnet, dass** es einen pH-Wert im Bereich von 2 bis 7 hat.

12. Transparentes Haar färbendes Konditionierungsmittel nach einem der vorhergehenden Ansprüche, **dadurch gekennzeichnet, dass** es eine Viskosität zwischen 1,000 mPa.s bis 70,000 mPa.s hat, gemessen mit einem Brookfield Viscosimeter bei 20°C, zum Beispiel mit Spindel 4 bei 10 rpm.

## Revendications

1. Agent de soin de coloration capillaire transparent **caractérisé en ce qu'**il comprend du polyquaternium-37 en tant qu'épaississant et stabilisant, au moins un colorant capillaire cationique à action directe, en tant qu'agents de restructuration capillaire, une céramide selon la formule 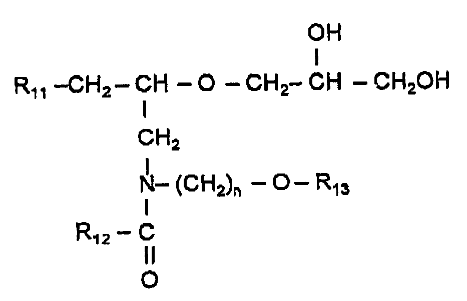 dans laquelle R¹¹ et R¹² représentent, indépendamment l'un de l'autre, un groupe alkyle ou alcényle avec 10 à 22 atomes de carbone, R¹³ représente un groupe méthyle, éthyle, n-propyle ou isopropyle et n représente un nombre compris entre 1 et 6, de préférence 2 ou 3 à une concentration de 0,01 à 2 % en poids, des acides gras avec 10 à 24 atomes de carbone à une concentration de 0,01 à 2,5 % en poids et du phytostérol
et exempt d'alcool gras de sorte qu'il n'est pas sous la forme d'une émulsion, et toutes les concentrations sont calculées par rapport à la composition totale.

2. Agent de soin de coloration capillaire transparent selon la revendication 1, **caractérisé en ce qu'**il comprend du polyquaternium 37 en tant qu'épaississant et stabilisant à une concentration comprise entre 0,01 et 5 % en poids calculée par rapport à la composition totale.

3. Agent de soin de coloration capillaire transparent selon les revendications 1 et 2, **caractérisé en ce qu'**il comprend au moins un colorant capillaire cationique à action directe à une concentration comprise entre 0,001 et 5 % en poids calculée par rapport à la composition totale.

4. Agent de soin capillaire transparent selon l'une quelconque des revendications précédentes, **caractérisé en ce qu'**il comprend des solubilisants choisis parmi des tensioactifs non ioniques, cationiques et amphotères/zwittérioniques.

5. Agent de soin capillaire transparent selon l'une quelconque des revendications précédentes, **caractérisé en ce qu'**il comprend de plus des principes actifs choisis parmi des produits restructurants pour cheveux, des produits hydratants et des extraits végétaux naturels.

6. Agent de soin capillaire transparent selon l'une quelconque des revendications précédentes, **caractérisé en ce qu'**il comprend des solvants organiques en tant que solubilisant et/ou agent améliorant la pénétration.

7. Agent de soin capillaire transparent selon l'une quelconque des revendications précédentes, **caractérisé en ce qu'**il comprend des polymères cationiques en tant qu'agents de soin supplémentaires.

8. Agent de soin capillaire transparent selon l'une quelconque des revendications précédentes, **caractérisé en ce qu'**il comprend des polymères non ioniques.

9. Agent de soin capillaire transparent selon l'une quelconque des revendications précédentes, **caractérisé en ce qu'**il est exempt de l'un quelconque parmi les polymères anioniques.

10. Agent de soin capillaire transparent selon l'une quelconque des revendications précédentes, **caractérisé en ce qu'**il comprend des acides organiques et/ou inorganiques et/ou leurs mélanges et/ou sels.

11. Agent de soin capillaire transparent selon l'une quelconque des revendications précédentes, **caractérisé en ce qu'**il possède un pH dans la gamme de 2 à 7.

12. Agent de soin capillaire transparent selon l'une quelconque des revendications précédentes, **caractérisé en ce qu'**il possède une viscosité comprise entre 1 000 mPa.s et 70 000 mPa.s mesurée au moyen d'un viscosimètre Brookfield à 20°C avec, par exemple, une aiguille n° 4 à 10 tr/mn.
